(19) Europäisches Patentamt
European Patent Office
Office européen des brevets

(11) Veröffentlichungsnummer: **0 030 295**
**B1**

(12) # EUROPÄISCHE PATENTSCHRIFT

(45) Veröffentlichungstag der Patentschrift: **25.05.83**

(51) Int. Cl.³: **C 07 H 15/24**

(21) Anmeldenummer: **80107162.2**

(22) Anmeldetag: **18.11.80**

(54) **Verfahren zur Herstellung von 4'-Epidaunorubicin, 3',4'-Diepidaunorubicin, hierbei entstehende Zwischenprodukte sowie deren Verwendung.**

(30) Priorität: **22.11.79 GB 7940457**

(43) Veröffentlichungstag der Anmeldung: **17.06.81 Patentblatt 81/24**

(45) Bekanntmachung des Hinweises auf die Patenterteilung: **25.05.83 Patentblatt 83/21**

(84) Benannte Vertragsstaaten: **BE CH DE FR GB LI NL SE**

(56) Entgegenhaltungen:
**FR-A-2 264 555**
**FR-A-2 309 234**
**FR-A-2 320 306**
**US-A-4 039 663**

(73) Patentinhaber: **FARMITALIA CARLO ERBA S.p.A., Via Carlo Imbonati n.24, I-20159 Milan (IT)**

(72) Erfinder: **Suarato, Antonino, Via Pacini n.21, Mailand (IT)**
Erfinder: **Penco, Sergio, Via Crimea n.13, Mailand (IT)**
Erfinder: **Arcamone, Federico, Via 4 Novembre n.26, Nerviano (Mailand) (IT)**

(74) Vertreter: **Hansen, Bernd, Dr.rer.nat. et al, Hoffmann . Eitle & Partner Patentanwälte Arabellastrasse 4, D-8000 München 81 (DE)**

**EP 0 030 295 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von 4'-Epidaunorubicin, 3',4'-Diepidaunorubicin, hierbei entstehende Zwischenprodukte sowie deren Verwendung

Die Erfindung bezieht sich auf ein neues Verfahren zur Herstellung von 4'-Epidaunorubicin und 3',4'-Diepidaunorubicin, hierbei entstehende Zwischenprodukte, sowie deren Verwendung. Die genannten Verbindungen sind bei der Behandlung bestimmter Tumore bei Tieren verwendbar und wurden bereits beschrieben: 4'-Epidaunorubicin in US-PS 4 039 663 und 3', 4'-Diepidaunorubicin in der US-PS 4 112 076.

Die Erfindung bezieht sich somit auf ein neues Verfahren zur Herstellung von Daunorubicinanaloga der allgemeinen Formel

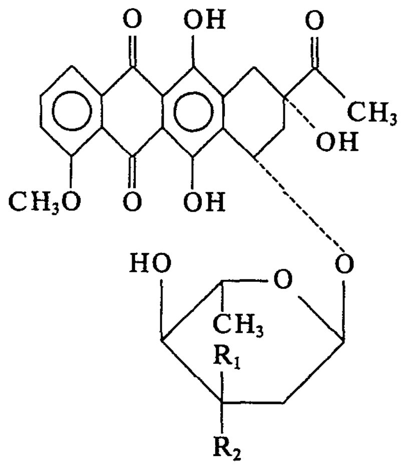

worin

a) $R_1$ Wasserstoff und $R_2$ eine $NH_2$-Gruppe, oder
b) $R_1$ eine $NH_2$-Gruppe und $R_2$ Wasserstoff bedeuten.

Das Verfahren läuft über neue Zwischenprodukte, nämlich ein 4'-keto-N-geschütztes Daunorubicin (IIIa, b — IVa, b).

Das Verfahren gemäß der Erfindung ist dadurch gekennzeichnet, daß man ein N-geschütztes Daunorubicin der Formel

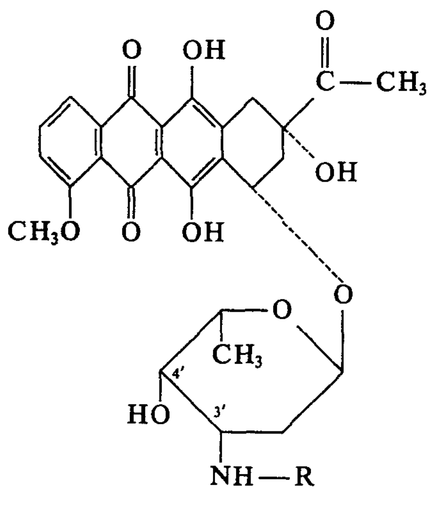

(I)

wobei R eine geeignete Schutzgruppe darstellt, in wasserfreiem Lösungsmittel durch Behandlung mit Dimethylsulfoxid, aktiviert durch Trifluoracetanhydrid, einer oxidativen Reaktion der C-4'-Hydroxylgruppe unterwirft, wobei nach Basischmachen der Reaktionslösung mit einer Base, ein N-geschütztes

4′-Keto-daunorubicin der Formel

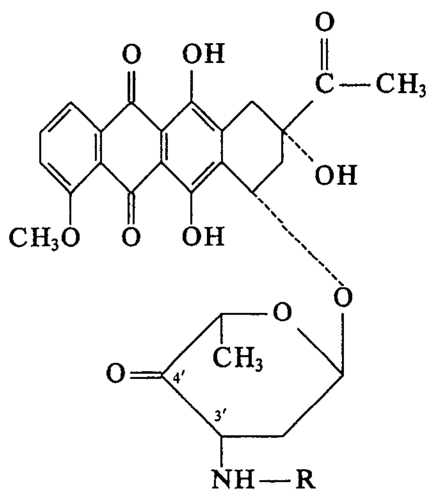

(IIIa)

erhalten wird, das mit Natriumborhydrid zu einem N-geschützten 4′-Epi-daunorubicin der Formel

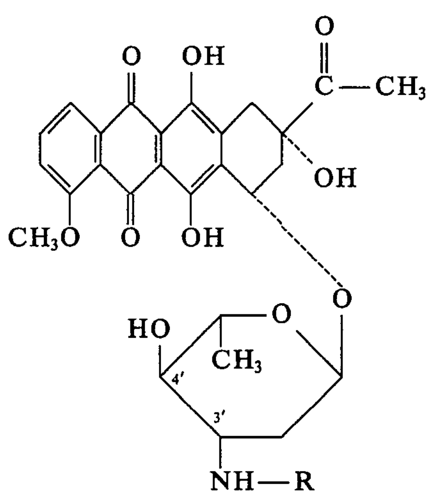

(Va)

umgesetzt wird, aus welchem nach milder alkalischer Hydrolyse der N-Schutzgruppe 4′-Epi-daunorubicin gewonnen und anschließend durch Behandlung mit methanolischem Halogenwasserstoff als Hydrohalogenid (VII) isoliert wird, oder die Verbindung (IIIa), gelöst in Halogenkohlenwasserstoff durch eine Silikagelsäure, gepuffert auf etwa pH 7, filtriert wird, wobei eine Mischung der Verbindung (IIIa) und von N-geschütztem 3′-Epi-4′-keto-daunorubicin der Formel

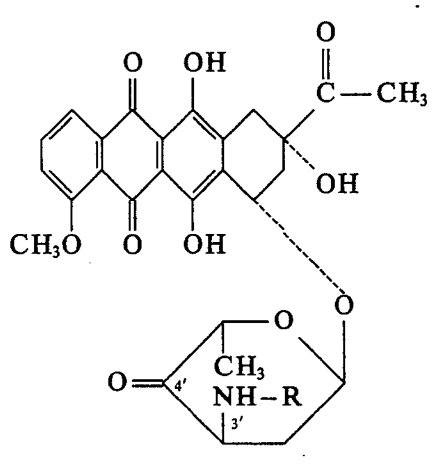

(IVa)

erhalten wird, worauf diese epimere Mischung mit Natriumborhydrid stereospezifisch reduziert wird,

wobei eine Mischung von N-geschütztem 4'-Epi-daunorubicin (Va) und N-geschütztem 3',4'-Epi-daunorubicin der Formel

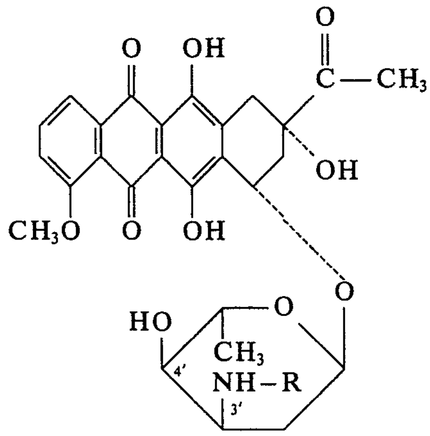

(VIa)

erhalten wird, worauf die reine Verbindung (VIa) aus dieser Mischung durch Chromatografie abgetrennt und anschließend die N-Schutzgruppe am Zuckerteil hydrolysiert wird, wobei die freie Glycosidbase 3',4'-Epidaunorubicin erhalten wird, die durch Behandlung mit methanolischem Halogenwasserstoff als entsprechendes Hydrohalogenid (VIII) isoliert wird.

Nach einer bevorzugten Ausführungsform der Erfindung wird das Verfahren derart ausgeführt, daß man N-Trifluoracetyldaunorubicin der Formel

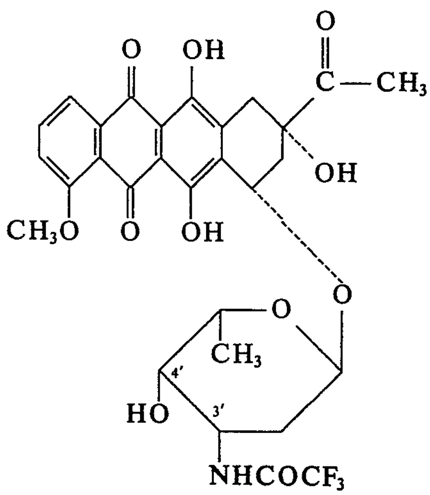

(I)

in wasserfreiem Lösungsmittel durch Behandlung mit Dimethylsulfoxid, aktiviert durch Trifluoracetanhydrid, einer oxidativen Reaktion der C-4'-Hydroxylgruppe unterwirft, wobei nach Basischmachen der Reaktionsmischung mit einer Base 4'-Keto-N-trifluoracetyldaunorubicin der Formel

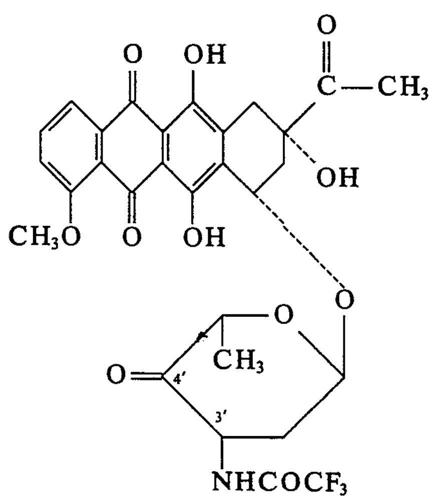

(IIIa)

erhalten wird, das mit Natriumborhydrid zu 4'-Epi-N-trifluoracetyldaunorubicin der Formel

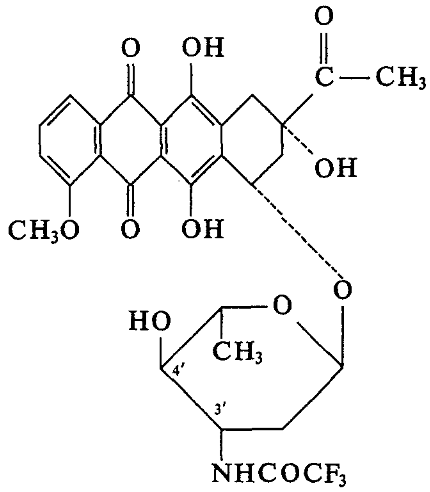

(Va)

umgesetzt wird, aus welchem nach milder alkalischer Hydrolyse der N-Trifluoracetylgruppe 4'-Epidaunorubicin gewonnen und anschließend durch Behandlung mit methanolischem Halogenwasserstoff als Hydrohalogenid (VII) isoliert wird, oder aber die Verbindung (IIIa), gelöst in Halogenkohlenwasserstoff durch eine Silikagelsäule, gepuffert auf etwa pH 7, filtriert wird, wobei eine Mischung der Verbindung (IIIa) und von 3'-Epi-4'-keto-N-trifluoracetyldaunorubicin der Formel

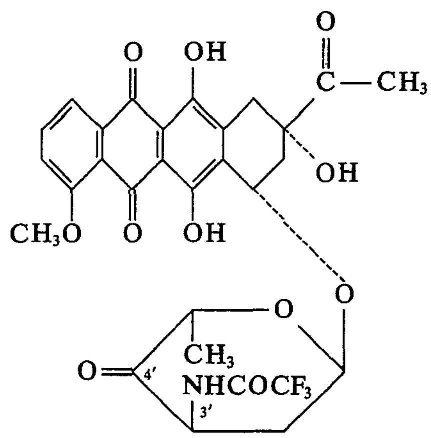

(IVa)

erhalten wird, worauf diese epimere Mischung mit Natriumborhydrid stereospezifisch reduziert wird, wobei eine Mischung von 4'-Epi-N-trifluoracetyldaunorubicin (Va) und 3',4'-Epi-trifluoracetyldaunorubicin der Formel

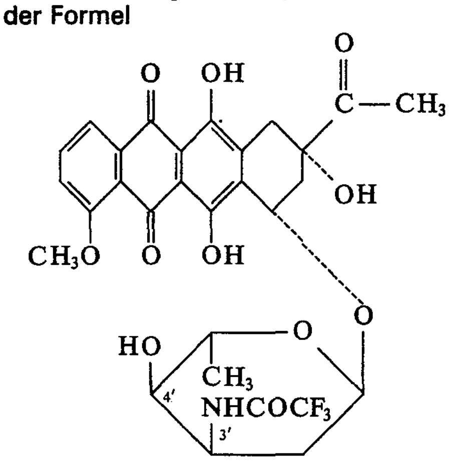

(VIa)

erhalten wird, worauf die reine Verbindung (VIa) aus dieser Mischung durch Chromatografie abgetrennt und anschließend die N-Trifluoracetylgruppe am Zuckerteil hydrolysiert wird, wobei die freie Glycosidbase 3',4'-Epidaunorubicin erhalten wird, die durch Behandlung mit methanolischem Halogenwasserstoff als entsprechendes Hydrohalogenid (VIII) isoliert wird.

Nach dem erfindungsgemäßen Verfahren, das im folgenden schematisch in einer bevorzugten Ausführungsform dargestellt ist, wird N-geschütztes Daunorubicin (I, II) in 4'-Epi- und

3',4'-Diepidaunorubicin übergeführt:

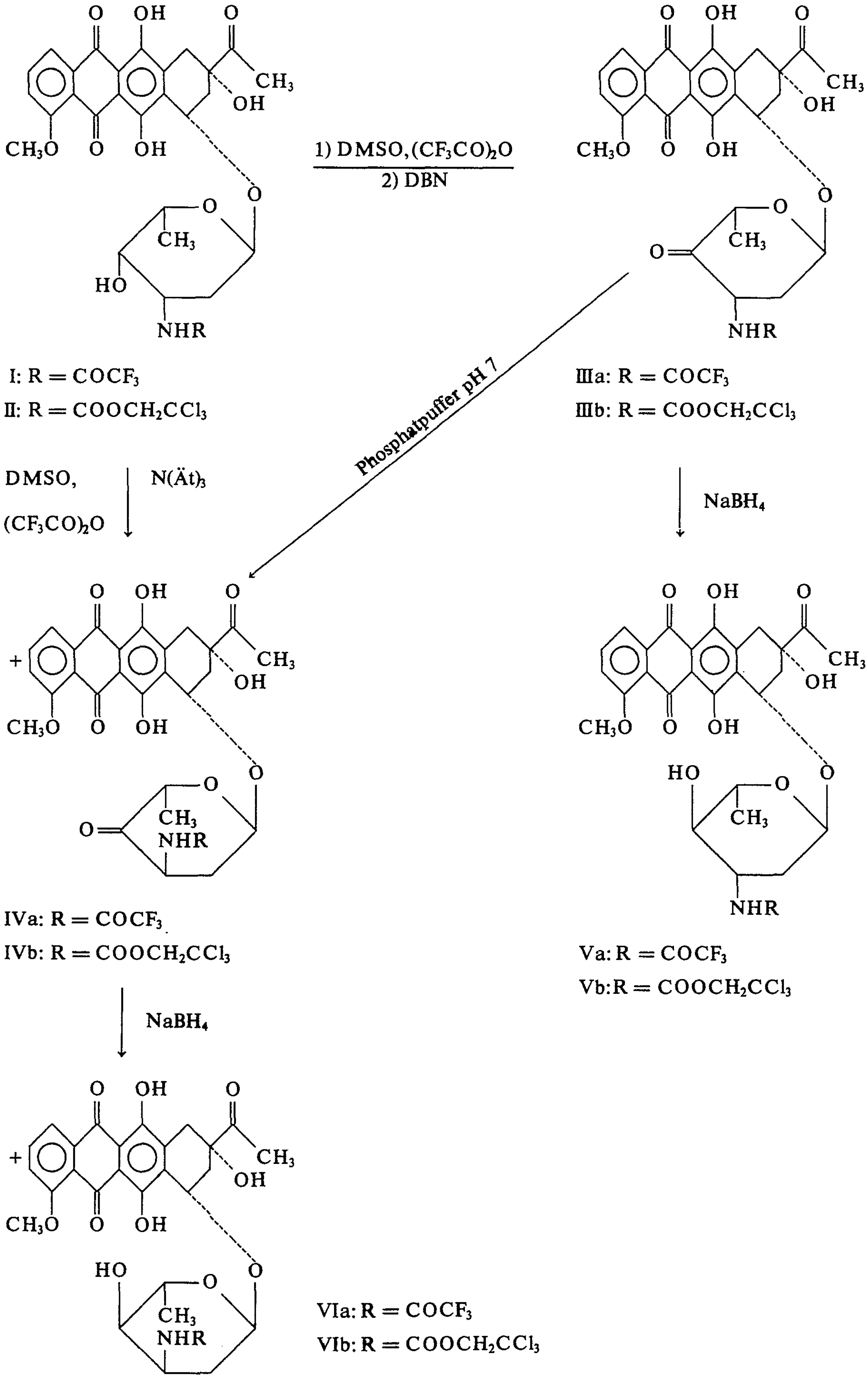


I: $R = COCF_3$
II: $R = COOCH_2CCl_3$

IIIa: $R = COCF_3$
IIIb: $R = COOCH_2CCl_3$

IVa: $R = COCF_3$
IVb: $R = COOCH_2CCl_3$

Va: $R = COCF_3$
Vb: $R = COOCH_2CCl_3$

VIa: $R = COCF_3$
VIb: $R = COOCH_2CCl_3$

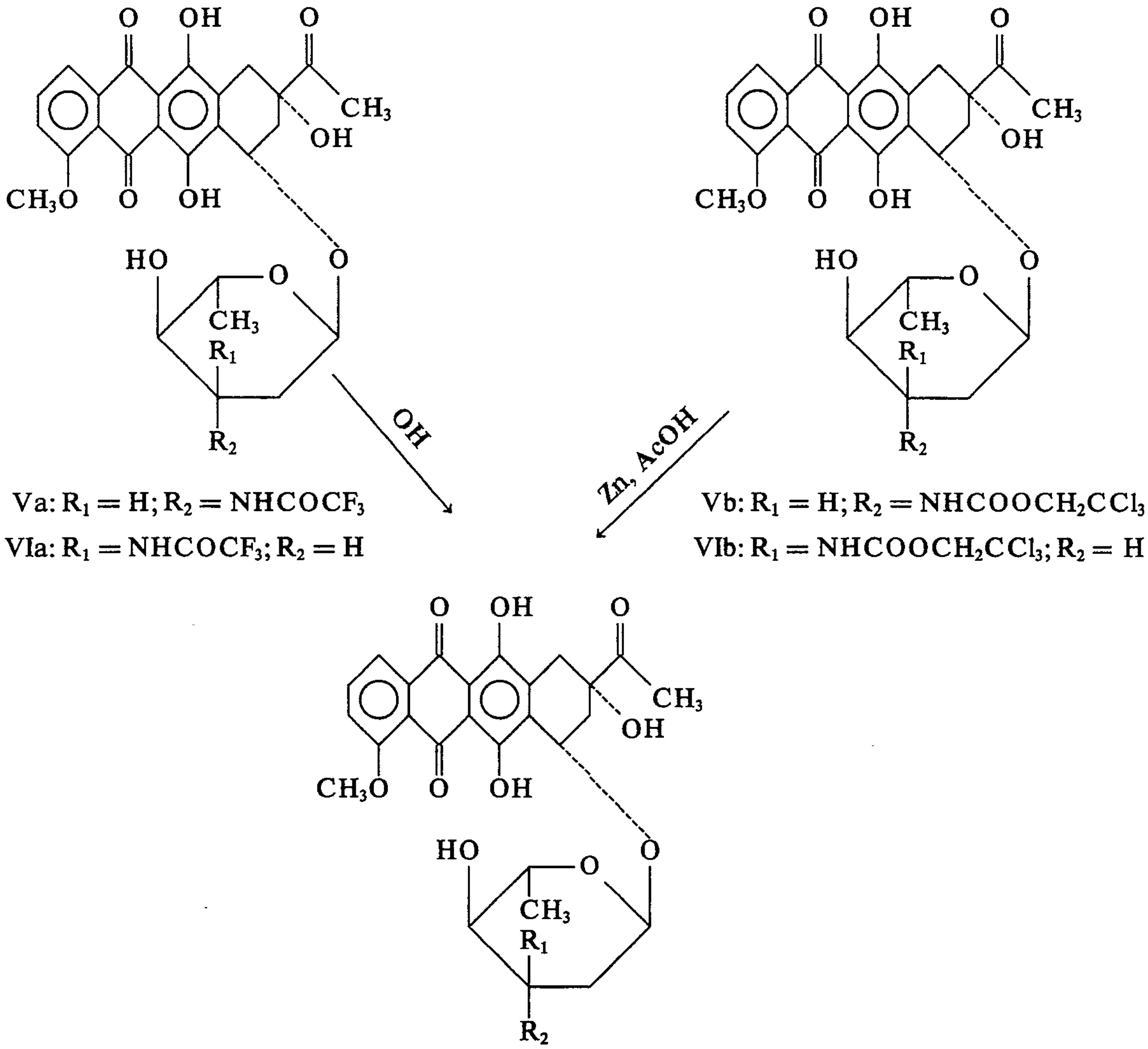


Va: $R_1 = H; R_2 = NHCOCF_3$
VIa: $R_1 = NHCOCF_3; R_2 = H$

Vb: $R_1 = H; R_2 = NHCOOCH_2CCl_3$
VIb: $R_1 = NHCOOCH_2CCl_3; R_2 = H$

VII: $R_1 = H; R_2 = NH_2$
VIII: $R_1 = NH_2; R_2 = H$

Um die Oxidation der Hydroxylgruppe bei C-4′ von Daunorubicin zu bewirken, ist es notwendig, die Aminogruppe des Zuckerteils mit einer geeigneten Schutzgruppe zu schützen, beispielsweise einem Acylderivat oder einer Schiff'schen Base, besonders bevorzugt sind die Trifluoracetyl- und die Trichlorethoxycarbonylgruppe (entsprechend Verbindung I oder II). Das N-geschützte Daunorubicin (I und II) wird einer oxidativen Reaktion unterworfen, die vorteilhaft durch aktiviertes Dimethylsulfoxid durchgeführt wird (K. Omura und D. Swern, Tetrahedron 1978, 34, 1651 – 1660). Es ist erwähnenswert, daß die Verwendung einer Base, wie 1,5-Diazabicyclo[4,3,O]non-5-en (DBN), beim Schritt des Basischmachens der Oxidation die Ketone (IIIa, b) in hoher Ausbeute ergibt, während die Verwendung von Triethylamin das asymmetrische Zentrum C-3′ selektiv beeinflußt, wobei eine Mischung von Ketonepimeren bei C-3′ (IIIa, b — IVa, b) im ungefähren Verhältnis von 1 : 1 erhalten wird. Außerdem kann die oben erwähnte Epimerisierung durch Behandlung der Ketone (IIIa, b) mit Silikagel, mit Phosphatpuffer auf pH 7 gepuffert, erzielt werden. Die selektive und stereospezifische Reduktion der Carbonylfunktion im Zuckerteil der Verbindungen (IIIa, b — IVa, b) ergibt die N-geschützten Glycoside (Va, b) bzw. (VIa, b) mit L-Arabino- und L-Ribokonfiguration. Die Hydrolyse der N-Schutzgruppe ergibt die Glycoside (VII) und (VIII), die als Hydrochloride isoliert werden.

Im folgenden werden bevorzugte Ausführungsformen des erfindungsgemäßen Verfahrens beschrieben:

Das Ausgangsmaterial für das erfindungsgemäße Verfahren ist das bekannte Daunorubicin. Dessen N-Trifluoracetylderivat (I) wird leicht auf die in der US-PS 3 803 124 beschriebene Weise hergestellt, indem mit Trifluoracetanhydrid in Anwesenheit eines organischen Lösungsmittels umgesetzt wird, worauf O-Trifluoracetylgruppen mit Methanol hydrolysiert werden. N-Trichlorethoxycarbonyldaunorubicin (II) wird durch Behandlung von Daunorubicin, als freie Base, in wäßriger Lösung mit Trichlorethoxycarboxychlorid hergestellt. Die Oxidation der Hydroxylgruppen der Verbindung (I) oder

(II) bei C-4′ wird mit DMSO, aktiviert durch Trifluoracetanhydrid, bewirkt. Das Reagens wird in wasserfreiem Methylenchlorid, das in geeigneter Weise auf −50 bis −70°C gekühlt wurde, hergestellt. Das Substrat (I oder II), in wasserfreiem Methylenchlorid gelöst, wird zu der Suspension des Reagens zugegeben, wobei die Temperatur bei −65°C gehalten wird, um das Dimethylalkoxysulfoniumsalz zu bilden. Die Bildung dieses Salzes kann mittels Dünnschichtchromatografie beobachtet werden: das Verschwinden des Ausgangsmaterials ist nach etwa 30 Minuten beendet. Dann wird eine Base, wie 1,5-Diazabicyclo[4,3,O]non-5-en (DBN), rasch zu der bei −65°C gehaltenen Reaktionsmischung zugesetzt. Wenn das Zusetzen der Base beendet ist (1 Minute), wird die Reaktionsmischung mit Essigsäure neutralisiert und gekühlt, indem sie in ein organisches Lösungsmittel, wie Methylenchlorid, gegossen wird; darauf wird die Lösung mit Wasser, verdünnter wäßriger Natriumbicarbonatlösung und Wasser gewaschen. Das Abdampfen des Lösungsmittels bis zur Trockene ergibt das Keton (IIIa, b) in quantitativer Ausbeute. Andererseits ergibt die Verwendung von Triethylamin anstelle von DBN die Mischung der Ketone (IIIa, b — IVa, b) in einem ungefähren Verhältnis von 1 : 1. Die Reduktion wird in einem mit Wasser mischbaren organischen Lösungsmittel, wie Methanol oder Dioxan, mit $NaBH_4$ durchgeführt, wobei 10 Minuten lang auf −10°C gekühlt wird. Unter diesen Bedingungen wird die Reduktion der Carbonylgruppe in der Seitenkette des Chromophors minimal gehalten und die axiale Bindung des Hydridions wird bevorzugt, wobei die entsprechenden Glycoside mit äquatorialer Hydroxylgruppe bei C-4′ erhalten werden. Wenn die Reduktion beendet ist, wird die Reaktionsmischung mit Methylenchlorid verdünnt und mit Wasser, verdünnter HCl, Wasser, verdünntem $NaHCO_3$ und wieder Wasser gewaschen. Bei Abdampfen des Lösungsmittels wird die rohe Verbindung (Va, b) oder eine Mischung der Verbindungen (Va, b — VIa, b) erhalten, die durch Chromatografie an einer Kieselsäuresäule gereinigt wird. Durch milde alkalische Behandlung wird die N-Trifluoracetylgruppe aus der Verbindung (Va — VIa) entfernt. Andererseits wird die Trichlorethoxycarboxyamidogruppe durch Behandlung der Verbindung (Vb — VIb) mit Zn-Staub in Anwesenheit von Essigsäure und Ethanol gespalten.

Es ist möglich, durch nachfolgende Behandlung der Verbindung (VII) und (VIII) gemäß der in der US-PS 4 058 519 bzw. der US-PS 4 112 076 beschriebenen Verfahren 4′-Epidoxorubicin und 3′,4′-Diepidoxorubicin herzustellen.

Im Rahmen der Erfindung werden auch die neuen Zwischenprodukte der Formeln IIIa, b und IVa, b beansprucht.

4′-Keto-N-trifluoracetyldaunorubicin (IIIa),
4′-Keto-N-Trichlorethoxycarbonyldaunorubicin (IIIb),
3′-Epi-4′-keto-N-trifluoracetyldaunorubicin (IVa),
3′-Epi-4′-keto-N-trichlorethoxycarbonyldaunorubicin (IVb).

Des weiteren wird die Verwendung solcher Zwischenprodukte zur Herstellung von 4′-Epidaunorubicin und 3′,4′-Diepidaunorubicin erfindungsgemäß in Betracht gezogen.

Die folgenden Beispiele sollen die vorliegende Erfindung näher erläutern, ohne daß diese hierauf beschränkt sein soll.

## Beispiel 1

### 4′-Keto-N-trifluoracetyldaunorubicin (IIIa)

Zu einer Mischung von 60 ml Methylenchlorid und 5 ml wasserfreiem Dimethylsulfoxid, unter −60°C gekühlt, wurde während eines Zeitraumes von 15 Minuten eine Lösung von 4 ml Trifluoracetanhydrid in 10 ml wasserfreiem Methylenchlorid zugegeben. Während der Zugabe bildete sich ein weißer Niederschlag. Nach 15 Minuten bei −60°C wurde eine Lösung von 6,25 g N-Trifluoracetyldaunorubicin (I) in 40 ml Methylenchlorid tropfenweise während eines Zeitraumes von 15 Minuten zu der Mischung zugesetzt. Die Reaktionsmischung, die 30 Minuten lang bei −60°C gerührt worden war, wurde rasch mit 9 ml 1,5-Diazabicyclo[4,3,O]non-5-3n (DBN) behandelt, wobei die Temperatur bei −60°C gehalten wurde. Nach 1 Minute wurde die Reaktionsmischung mit der stöchiometrischen Menge Essigsäure neutralisiert und in 300 ml Methylenchlorid gegossen. Die organische Phase wurde mit wäßriger 0,1 n HCl, einer wäßrigen $NaHCO_3$-Lösung und Wasser gewaschen. Die organische Lösung, über wasserfreiem $Na_2SO_4$ getrocknet, wurde zur Trockene eingedampft, wobei die rohe Verbindung (IIIa) erhalten wurde, die durch Chromatografie auf einer Silikagelsäule unter Verwendung einer Mischung aus Chloroform — Aceton (98 : 2, V/V) als Eluierungsmittel 4,8 g der Verbindung (IIIa) ergab:

FDMS [M+]: 621
NMR ($CDCl_3$): 1,37 (d, $CH_3$-C-5′), 2,38 (s, $CH_3CO$), 3,95 (s, $CH_3O$), 4,78 (breites q, C-5′-H), 5,20 (breites s, C-7-H), 5,58 (breites s, C-1′-H), 12,93 und 13,83 $\delta$ (s, phenolische Protonen.

Beispiel 2

4'-Epidaunorubicin (VII)

Eine Lösung von 1,5 g der Verbindung (IIIa) in 150 ml Methanol wurde auf −10° C gekühlt und mit 0,035 g $NaBH_4$, gelöst in 5 ml Methanol, behandelt. Nach 10 Minuten war die Reduktion beendet, worauf die Reaktionsmischung mit wäßriger 0,1 n HCl neutralisiert, im Vakuum auf ein geringes Volumen (30 ml) eingedampft und mit 200 ml Methylenchlorid verdünnt wurde. Die mit Wasser gewaschene organische Lösung wurde über $Na_2SO_4$ getrocknet und zur Trockene eingedampft. Der Rückstand, rohes 4'-Epi-N-trifluoracetyldaunorubicin (Va) wurde in 50 ml 0,1 n wäßrigem Natriumhydroxid gelöst. Die erhaltene Lösung wurde nach Stehenlassen während 30 Minuten bei 5° C mit 0,1 n wäßrigem Chlorwasserstoff behandelt, um den pH-Wert auf 4,5 einzustellen, und mit Chloroform extrahiert, um die Algycone zu entfernen. Dann wurde die wäßrige Lösung auf einen pH-Wert von 8,6 eingestellt und wiederholt mit Chloroform extrahiert. Die vereinigten Extrakte wurden über wasserfreiem $Na_2SO_4$ getrocknet, auf ein geringes Volumen eingedampft und mit 0,1 n methanolischem Chlorwasserstoff angesäuert, um die Kristallisation von 4'-Epidaunorubicinhydrochlorid (VII) zu ermöglichen;

Fp.: 199 bis 201° C,
$[\alpha]_D$ +320° (c = 0,045 in MeOH) (siehe US-PS 4 039 663).

Beispiel 3

N-Trichlorethoxycarbonyldaunorubicin (II)

Eine wäßrige Lösung von 3 g Daunorubicinhydrochlorid in 180 ml Wasser wurde mit wäßriger 0,2 n NaOH auf einen pH-Wert von 10 eingestellt und anschließend unter Rühren mit einer Lösung von 2 g 2,2,2-Trichlorethylchlorformiat in 10 ml Aceton behandelt. Die Reaktionsmischung wurde durch Zusetzen von wäßriger 0,2 n NaOH bei pH 10 gehalten und nach 30 Minuten bei Raumtemperatur wurden 200 ml Methylenchlorid zugegeben. Die organische Phase wurde abgetrennt, zweimal mit je 100 ml Wasser gewaschen, über wasserfreiem $Na_2SO_4$ getrocknet und unter Vakuum zur Trockene eingedampft, wobei 3,1 g der Verbindung (II) erhalten wurden.

Dünnschichtchromatografie auf Kieselgelplatte $F_{254}$ Merck unter Verwendung des Systems $CHCl_3-(CH_3)_2CO$ (4 : 1, V/V): Rf = 0,4.

NMR ($CDCl_3$): 1,33 (d, $CH_3$-C-5'), 2,38 (s, $CH_3CO$), 4,03 (s, $CH_3O$), 4,63 (s, $O-CH_2-CCl_3$), 5,20 (breites s, C-7-H), 5,60 (breites s, C-1'-H), 13,13 und 13,92 $\delta$ (s, phenolische Protonen).

Beispiel 4

4'-Keto-N-trichlorethoxycarbonyldaunorubicin (IIIb)

7,04 g N-Trichlorethoxycarbonyldaunorubicin (II) wurden, wie in Beispiel 1 beschrieben, oxidiert, wobei 4'-Keto-N-trichlorethoxycarbonyldaunorubicin (IIIb) erhalten wurde:

FDMS [M+]: 699,
NMR ($CDCl_3$): 1,37 (d, $CH_3$-C-5'), 2,38 (s, $CH_3CO$), 4,05 (s, $CH_3O$), 4,63 (s, $O-CH_2-CCl_3$), 5,30 (breites s, C-7-H), 5,60 (breites s, C-1'-H), 13,12 und 13,95 $\delta$ (s, phenolische Protonen).

Beispiel 5

4'-Epidaunorubicin (VII)

Eine Lösung von 1,7 g der Verbindung (IIIb) in 200 ml Methanol wurde auf −10° C gekühlt und mit 0,035 g $NaBH_4$ behandelt. Nach 10 Minuten war die Reduktion beendet. Die Reaktionsmischung wurde unter Vakuum zur Trockene eingedampft und der Rückstand, in 200 ml Methylenchlorid gelöst, mit Wasser gewaschen. Der nach Abdampfen des Lösungsmittels erhaltene Rückstand wurde in 120 ml einer Mischung aus Ethanol−Methanol (3 : 1, V/V) gelöst und unter Rühren mit 1,5 g Zinkstaub, der vorher mit wäßrigem 0,1 n Chlorwasserstoff behandelt worden war, in Anwesenheit von Eisessig behandelt. Nach 60 Minuten bei Raumtemperatur wurde die Reaktionsmischung (auf Celite) abfiltriert, mit 300 ml Chloroform verdünnt und mit Wasser gewaschen. Die 4'-Epidaunorubicin (VII) enthaltende

rote wäßrige Lösung wurde abgetrennt und mit Chloroform extrahiert, um die Aglycone zu entfernen. Dann wurde die wäßrige Lösung auf pH 8,6 eingestellt und rasch mit Chloroform extrahiert. Die vereinigten Extrakte wurden über $Na_2SO_4$ getrocknet, auf ein geringes Volumen eingeengt und mit 0,1 n methanolischem Chlorwasserstoff auf pH 4,5 angesäuert, um die Kristallisation von 4'-Epidaunorubicinhydrochlorid (VII) zu ermöglichen.

Beispiel 6

4'-Keto-N-trifluoracetyldaunorubicin (IIIa) und
3'-Epi-4'-keto-N-trifluoracetyldaunorubicin (IVa)

Zu einer Mischung von 60 ml Methylenchlorid und 5 ml wasserfreiem Dimethylsulfoxid, gekühlt auf eine Temperatur unter −60° C, wurde eine Lösung von 4 ml Trifluoracetanhydrid in 10 ml wasserfreiem Methylenchlorid während eines Zeitraums von 15 Minuten zugegeben. Während der Zugabe bildete sich ein weißer Niederschlag. Nach 15 Minuten bei −60° C wurde eine Lösung von 6,25 g N-Trifluoracetyldaunorubicin (I) in 40 ml wasserfreiem Methylenchlorid tropfenweise zu der Mischung bei −60° C während eines Zeitraums von 15 Minuten zugesetzt. Dann wurde die Reaktionsmischung 30 Minuten bei −60° C gerührt und mit 9 ml Triethylamin behandelt, wobei die Temperatur bei −60° C gehalten wurde. Die Reaktionsmischung wurde in 300 ml Methylenchlorid gegossen und mit wäßriger 0,1 n HCl, wäßriger gesättigter $NaHCO_3$-Lösung und Wasser gewaschen. Die organische Lösung, über $Na_2SO_4$ getrocknet, wurde eingedampft, wobei eine rohe Mischung der Verbindungen (IIIa) und (IVa) erhalten wurde, die durch Chromatografie auf einer Silikagelsäule unter Verwendung der Mischung Chloroform – Aceton (98 : 2, V/V) als Eluierungsmittel gereinigt wurde, wobei 4,6 g der Mischung der Verbindungen (IIIa, IVa) in einem ungefähren Verhältnis von 1 : 1 erhalten wurden.

FDMS [$M^+$]: 621,
NMR ($CDCl_3$): 1,38 und 1,45 (zwei d [gleiche Intensität], $CH_3$-C-5'), 4,01 und 4,03 (zwei s [gleiche Intensität], $CH_3O$), 13,10 und 13,13 (zwei s [gleiche Intensität], C-11-OH) und 13,90 und 13,97 $\delta$ (zwei s [gleiche Intensität], C-6-OH).

Außerdem wurde die Mischung der Verbindungen (IIIa) und (IVa) im Verhältnis 1 : 1 durch einfaches Filtrieren einer chloroformischen Lösung der reinen Verbindung (IIIa) auf einer Silikagelschicht, mit Phosphatpuffer M/15 auf pH 7 gepuffert, erhalten.

Beispiel 7

4'-Keto-N-trichlorethoxycarbonyldaunorubicin (IIIb) und
3'-Epi-4'-keto-N-trichlorethoxycarbonyldaunorubicin (IVb)

7,04 g N-Trichlorethoxycarbonyldaunorubicin (II) wurden, wie in Beispiel 6 beschrieben, oxidiert, wobei eine Mischung von 4'-Keto-N-trichlorethoxycarbonyldaunorubicin (IIIb) und 3'-Epi-4'-keto-N-trichlorethoxycarbonyldaunorubicin (IVb) erhalten wurde.

Beispiel 8

3',4'-Diepidaunorubicin (VIII)

Eine Lösung von 6 g der Mischung von 4'-Keto-N-trifluoracetyldaunorubicin (IIIa) und 3'-Epi-4'-keto-N-trifluoracetyldaunorubicin, nach dem in Beispiel 6 beschriebenen Verfahren erhalten, in 600 ml Methanol wurde auf −10° C abgekühlt und mit 0,140 g $NaBH_4$, gelöst in 20 ml Methanol, behandelt. Nach 10 Minuten war die Reduktion beendet, worauf die Reaktionsmischung mit wäßriger 0,1 n HCl neutralisiert, unter Vakuum auf ein geringes Volumen (50 ml) eingedampft und mit 250 ml Methylenchlorid verdünnt wurde. Die organische Lösung wurde mit Wasser gewaschen, über $Na_2SO_4$ getrocknet und zur Trockene eingedampft. Der Rückstand (5,7 g) der Mischung von 4'-Epi-N-trifluoracetyldaunorubicin (Va) und 3',4'-Diepi-N-trifluoracetyldaunorubicin (VIa) im Verhältnis 1 : 1 wurde auf einer Silikagelsäule, mit Phosphatpuffer M/15 auf pH 7 gepuffert, unter Verwendung des Lösungsmittelsystems Chloroform – Aceton (96 : 4, V/V) als Eluierungsmittel chromatografiert. Es wurde ein gleicher Anteil an Verbindung (Va) und (VIa) erhalten, nämlich 1,9 g. Die Hydrolyse der N-Schutzgruppe der Verbindung (Va) und (VIa) erfolgte unter Anwendung des in Beispiel 2 beschriebenen Verfahrens, wobei in quantitativer Ausbeute 4'-Epidaunorubicinhydrochlorid (VII) und 3',4'-Diepidaunorubicin (VIII) erhalten wurden.

Fp.: 180 bis 181° C (Zersetzung)
$[\alpha]_D^{25} = +243,5°$ (c = 0,05 in Methanol) (siehe US-PS 4 112 076).

**Patentansprüche**

1. Verfahren zur Herstellung von 4'-Epidaunorubicin, 3',4'-Diepidaunorubicin bzw. deren Hydrohalogeniden der Formel

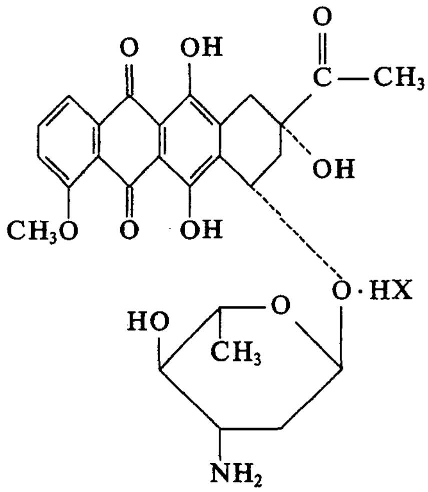

(VII)

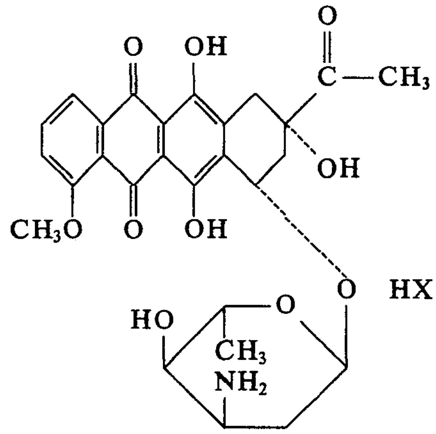

(VIII)

dadurch gekennzeichnet, daß man ein N-geschütztes Daunorubicin der Formel

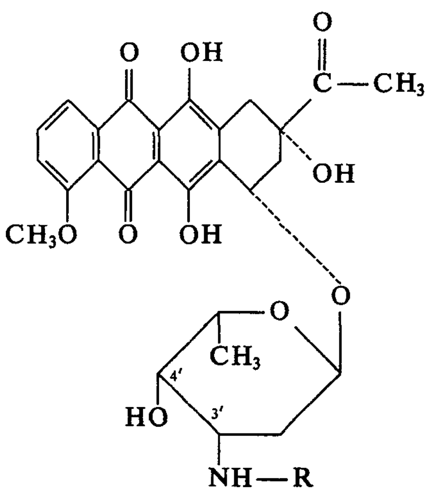

(I)

wobei R eine geeignete Schutzgruppe darstellt, in wasserfreiem Lösungsmittel durch Behandlung mit Dimethylsulfoxid, aktiviert durch Trifluoracetanhydrid, einer oxidativen Reaktion der C-4'-Hydroxylgruppe unterwirft, wobei nach Basischmachen der Reaktionslösung mit einer Base, ein N-geschütztes 4'-Keto-daunorubicin der Formel

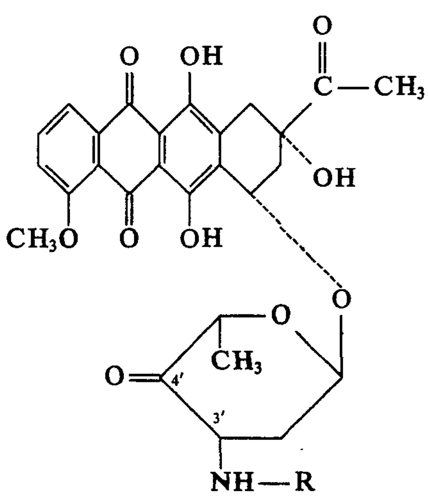

(IIIa)

erhalten wird, das mit Natriumborhydrid zu einem N-geschützten 4'-Epi-daunorubicin der Formel

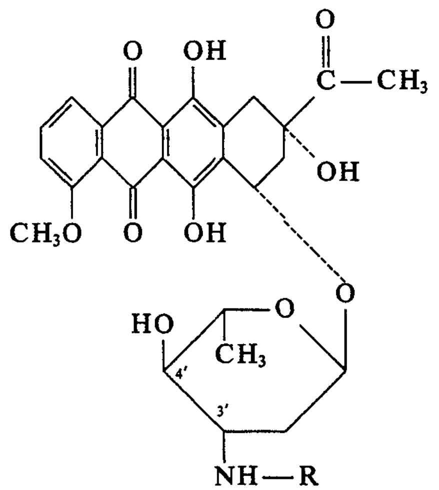

(Va)

umgesetzt wird, aus welchem nach milder alkalischer Hydrolyse der N-Schutzgruppe 4'-Epi-daunorubicin gewonnen und anschließend durch Behandlung mit methanolischem Halogenwasserstoff als Hydrohalogenid (VII) isoliert wird, oder
die Verbindung (IIIa), gelöst in Halogenkohlenwasserstoff durch eine Silikagelsäule, gepuffert auf etwa pH 7, filtriert wird, wobei eine Mischung der Verbindung (IIIa) und von N-geschütztem 3'-Epi-4'-keto-daunorubicin der Formel

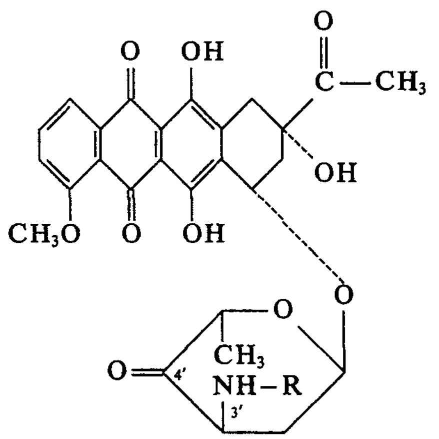

(IVa)

erhalten wird, worauf diese epimere Mischung mit Natriumborhydrid stereospezifisch reduziert wird, wobei eine Mischung von N-geschütztem 4'-Epi-daunorubicin (Va) und N-geschütztem 3',4'-Epi-daunorubicin der Formel

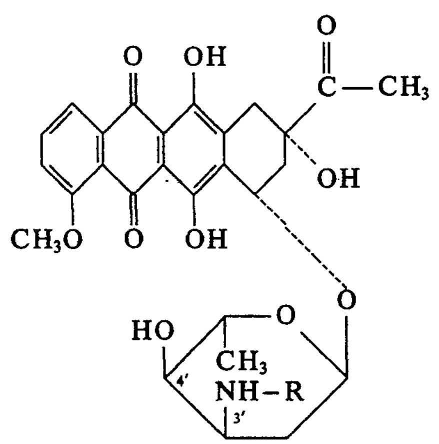

(VIa)

erhalten wird, worauf die reine Verbindung (VIa) aus dieser Mischung durch Chromatografie

abgetrennt und anschließend die N-Schutzgruppe am Zuckerteil hydrolysiert wird, wobei die freie Glycosidbase 3',4'-Epidaunorubicin erhalten wird, die durch Behandlung mit methanolischem Halogenwasserstoff als entsprechendes Hydrohalogenid (VIII) isoliert wird.

2. Verfahren gemäß Anspruch 1, dadurch gekennzeichnet, daß man N-Trifluoracetyldaunorubicin der Formel

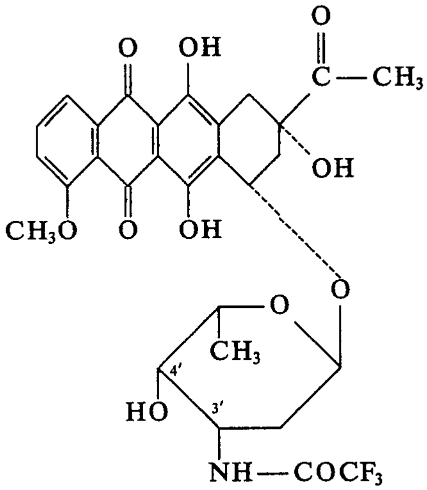

(I)

in wasserfreiem Lösungsmittel durch Behandlung mit Dimethylsulfoxid, aktiviert durch Trifluoracetanhydrid, einer oxidativen Reaktion der C-4'-Hydroxylgruppe unterwirft, wobei nach Basischmachen der Reaktionsmischung mit einer Base, 4'-Keto-N-trifluoracetyldaunorubicin der Formel

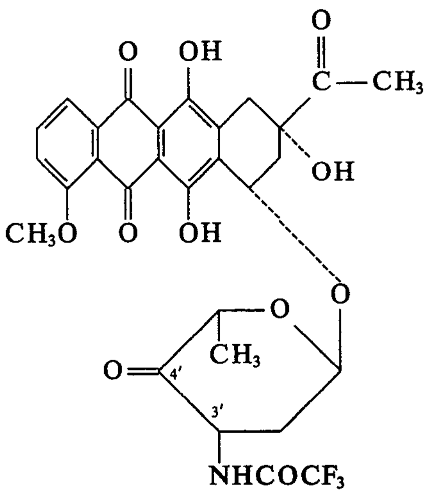

(IIIa)

erhalten wird, das mit Natriumborhydrid zu 4'-Epi-N-trifluoracetyldaunorubicin der Formel

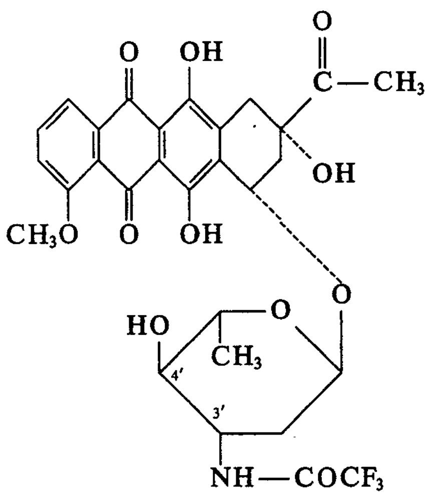

(Va)

umgesetzt wird, aus welchem nach milder alkalischer Hydrolyse der N-Trifluoracetylgruppe 4'-Epidaunorubicin gewonnen und anschließend durch Behandlung mit methanolischem Halogenwasserstoff als Hydrohalogenid (VII) isoliert wird, oder aber die Verbindung (IIIa), gelöst in Halogenkohlenwasserstoff durch eine Silikagelsäule, gepuffert auf etwa pH 7, filtriert wird, wobei eine Mischung der Verbindung (IIIa) und von 3'-Epi-4'-keto-N-trifluoracetyldaunorubicin der Formel

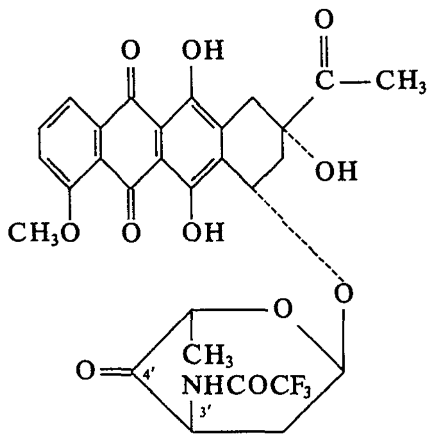

(IVa)

erhalten wird, worauf diese epimere Mischung mit Natriumborhydrid stereospezifisch reduziert wird, wobei eine Mischung von 4'-Epi-N-trifluoracetyldaunorubicin (Va) und 3',4'-Epi-N-trifluoracetyldaunorubicin der Formel

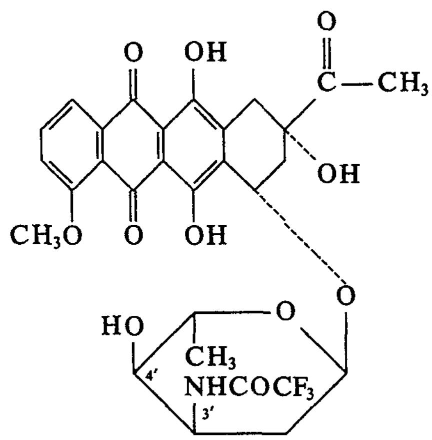

(VIa)

erhalten wird, worauf die reine Verbindung (VIa) aus dieser Mischung durch Chromatografie abgetrennt und anschließend die N-Trifluoroacetylgruppe am Zuckerteil hydrolysiert wird, wobei die freie Glycosidbase 3',4'-Epidaunorubicin erhalten wird, die durch Behandlung mit methanolischem Halogenwasserstoff als entsprechendes Hydrohalogenid (VIII) isoliert wird.

3. Verfahren nach Anspruch 1 oder 2, dadurch gekennzeichnet, daß man N-Trifluor-acetyldaunorubicin der Formel

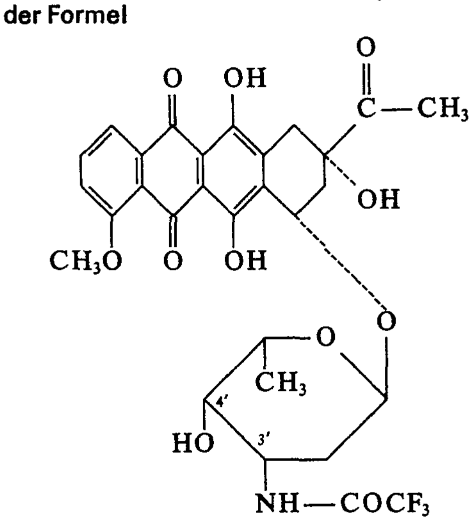

(I)

gelöst in wasserfreiem Methylenchlorid, durch Behandlung mit Dimethylsulfoxid, aktiviert durch Trifluoracetanhydrid, einer oxidativen Reaktion der C-4'-Hydroxylgruppe bei einer Temperatur von −65 bis −60°C unterwirft, wobei nach Basismachen der Reaktionsmischung mit einer Base, wie 1,5-Diazobicyclo[4,3,o]-non-5-en, das neue 4'-Keto-N-trifluoracetyldaunorubicin der Formel

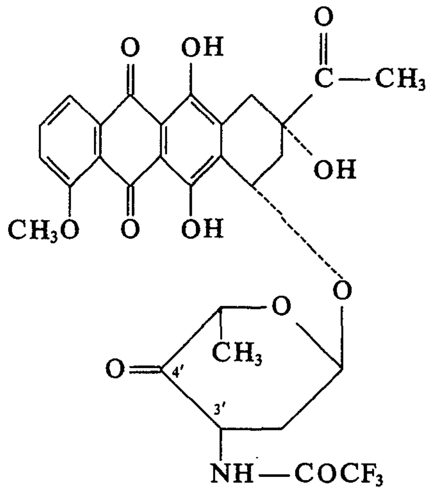

(IIIa)

erhalten wird, das, gelöst in Methanol, entweder bei einer Temperatur von −10°C mit Natriumborhydrid einer selektiven stereospezifischen Reduktion unterworfen wird, wobei 4'-Epi-N-trifluoracetyldaunorubicin der Formel

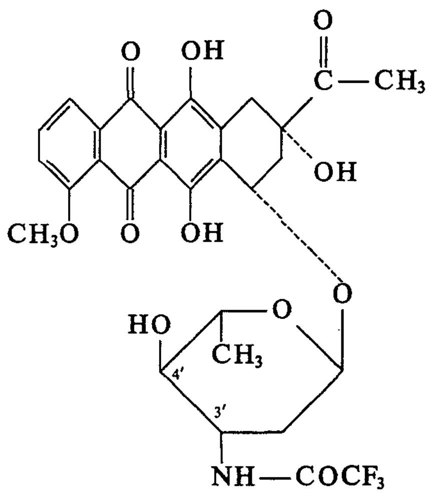

(Va)

erhalten wird, aus welchem nach milder alkalischer Hydrolyse der N-Trifluoracetylgruppe mit 0,1 n wäßrigem Natriumhydroxid 4'-Epidaunorubicin gewonnen und anschließend durch Behandlung mit methanolischem Chlorwasserstoff als Hydrochlorid (VII) isoliert wird, oder aber die Verbindung (IIIa), gelöst in Chloroform, durch eine Silikagelsäule, mit Phosphatpuffer M/15 auf pH 7 gepuffert, filtriert wird, wobei eine 1 : 1 epimere Mischung der Verbindung (IIIa) und des neuen 3'-Epi-4'-keto-N-trifluoracetyldaunorubicins der Formel

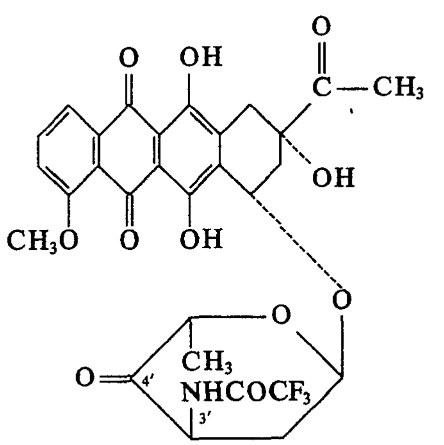

(IVa)

erhalten wird, worauf diese epimere Mischung mit Natriumborhydrid stereospezifisch reduziert wird, wobei eine 1 : 1 Mischung von 4'-Epi-N-trifluoracetyldaunorubicin (Va) und 3',4'-Epi-N-trifluoracetyldaunorubicin der Formel

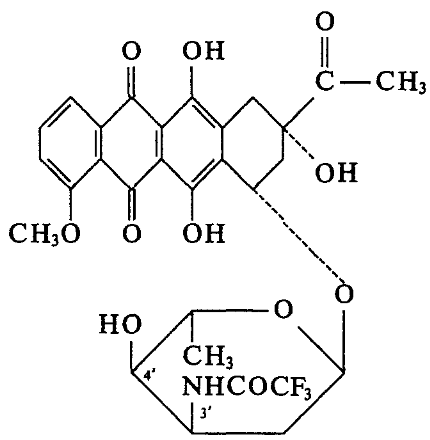

(VIa)

erhalten wird, worauf die reine Verbindung (VIa) aus dieser Mischung durch Chromatografie auf einer Silikagelsäule, mit Phosphatpuffer M/15 auf pH 7 gepuffert, unter Verwendung des Lösungsmittelsystems Chloroform-Aceton (96 : 4, V/V) abgetrennt und anschließend durch Behandlung mit 0,1 n Natriumhydroxid die N-Trifluoracetylgruppe am Zuckerteil hydrolysiert wird, wobei die freie Glycosidbase 3',4'-Epidaunorubicin erhalten wird, die schließlich durch Behandlung mit methanolischem Chlorwasserstoff als entsprechendes Hydrochlorid (VIII) isoliert wird.

4. 4'-Keto-N-trifluoracetyldaunorubicin.

5. 4'-Keto-N-trichlorethoxycarbonyldaunorubicin.

6. 3'-Epi-4'-keto-N-trifluoracetyldaunorubicin.

7. 3'-Epi-4'-keto-N-trichlorethoxycarbonyldaunorubicin.

8. Verwendung der Verbindungen gemäß einem oder mehreren der Ansprüche 4 bis 7 zur Herstellung von 4'-Epidaunorubicin und 3',4'-Diepidaunorubicin.

## Claims

1. A process for the preparation of 4'-epidaunorubicin, 3',4'-diepidaunorubicin and their hydrohalides according to the formulae

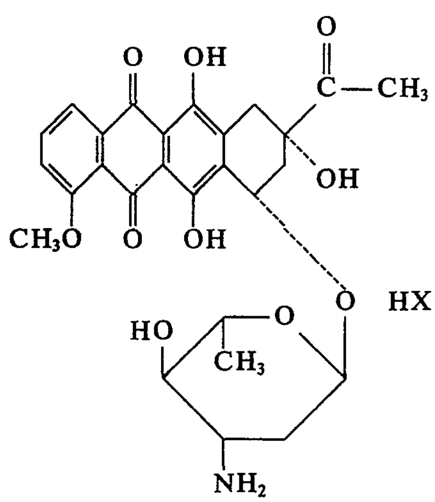

(VII)

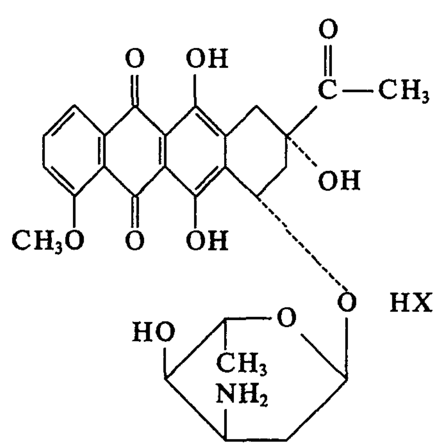

(VIII)

characterized in that an N-protected daunorubicin of the formula

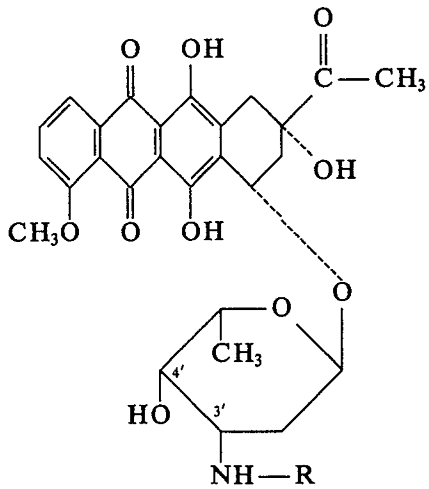

(I)

wherein R represents a suitable protective group, is subjected in an anhydrous solvent by treatment with dimethyl sulfoxide, activated by trifluoroacetic anhydride, to an oxidative reaction of the C-4′-hydroxyl group, thereby obtaining after alkalisation of the reaction solution with a base an N-protected 4′-keto-daunorubicin of the formula

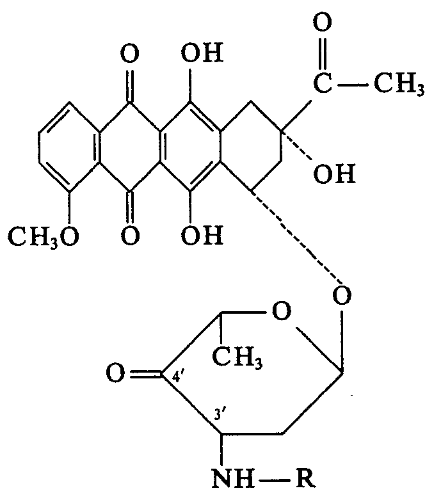

(IIIa)

which is reacted with sodium borohydride to an N-protected 4′-epi-daunorubicin of the formula

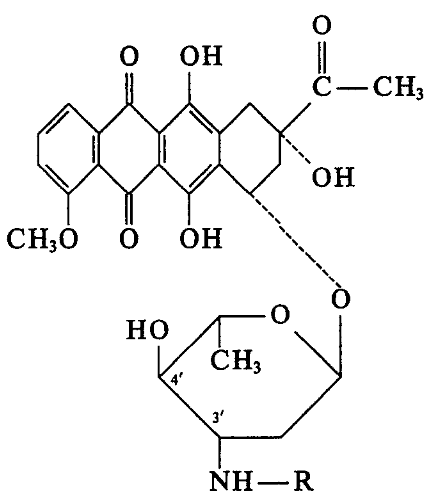

(Va)

from which after mild alkaline hydrolysis of the N-protective group there is obtained the compound 4'-epi-daunorubicin, and subsequently by treatment with methanolic hydrogen halide respective hydrohalide (VII) is isolated, or the compound (IIIa), dissolved in halogenated hydrocarbon, is filtered through a silica gel column, buffered to about pH 7, thereby obtaining a mixture of the compound (IIIa) and of N-protected 3'-epi-4'-keto-daunorubicin of the formula

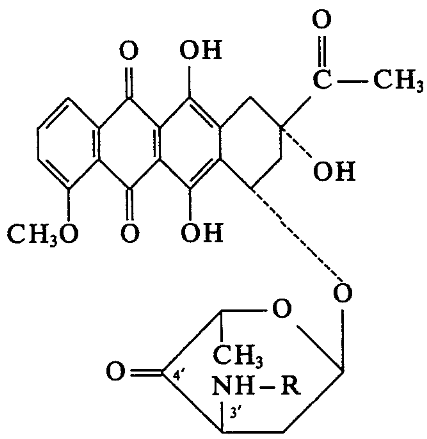

(IVa)

whereupon this epimeric mixture is reduced stereo-specifically with sodium borohydride, thereby obtaining a mixture of N-protected 4'-epi-daunorubicin (Va) and N-protected 3',4'-epi-daunorubicin of the formula

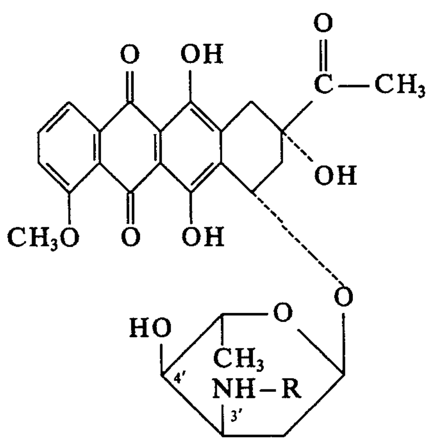

(VIa)

the pure compound (VIa) is separated from this mixture by chromatography, and subsequently the N-protective group at the sugar moiety is hydrolyzed, thereby obtaining the free glycoside base 3',4'-epidaunorubicin, which is isolated as respective hydrohalide (VIII) after treatment with methanolic hydrogen halide.

2. A process according to claim 1, characterized in that N-trifluoroacetyl-daunorubicin of the formula

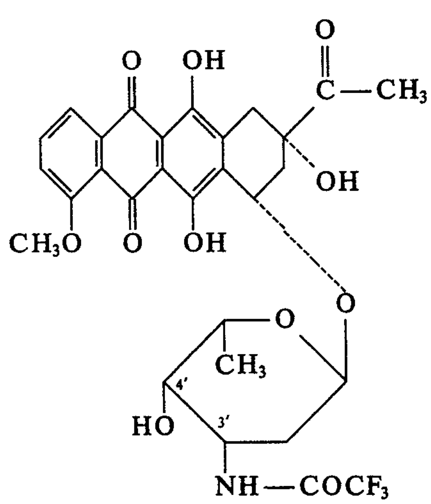

(I)

is subjected in an anhydrous solvent by treatment with dimenthyl sulfoxide, activated by trifluoro-acetic anhydride, to an oxidative reaction of the C-4'-hydroxyl group, thereby obtaining after alkalisation of the reaction mixture with a base the compound 4'-keto-N-trifluoroacetyldaunorubicin of the formula

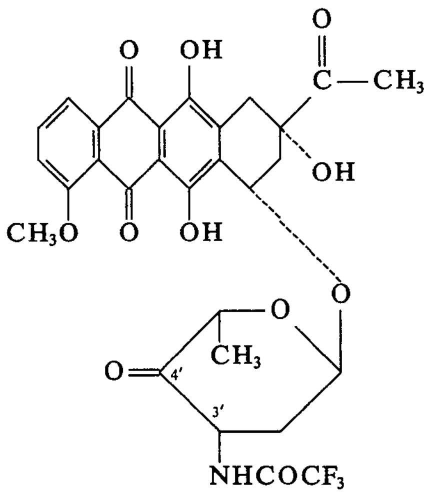

(IIIa)

which is reacted with sodium borohydride to 4'-epi-N-trifluoracetyl-daunorubicin of the formula

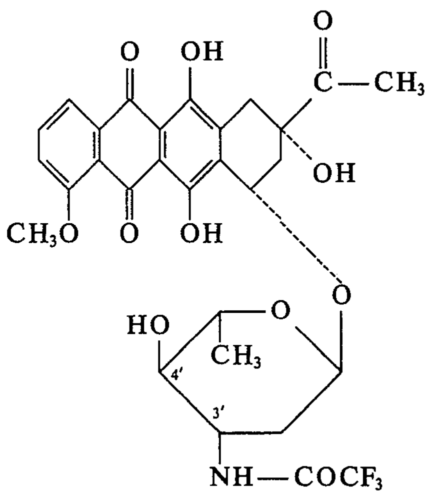

(Va)

from which after mild alkaline hydrolysis of the N-trifluoroacetyl group the compound 4'-epi-daunorubicin is obtained, and subsequently by treatment with methanolic hydrogen halide respective hydrohalide (VII) is isolated, or the compound (IIIa), dissolved in halogenated hydrocarbon, is filtered through a silica gel column, buffered to about pH 7, thereby obtaining a mixture of the compound (IIIa) and of 3'-epi-4'-keto-N-trifluoroacetyl-daunorubicin of the formula

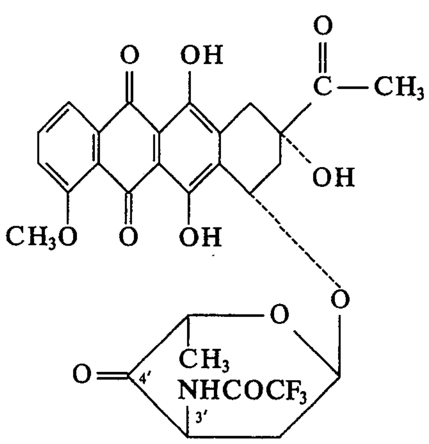

(IVa)

whereupon this epimeric mixture is reduced stereospecifically with sodium borohydride, thereby obtaining a mixture of 4'-epi-N-trifluoroacetyl-daunorubicin (Va) and 3',4'-epi-N-trifluoroacetyl-daunorubicin of the formula

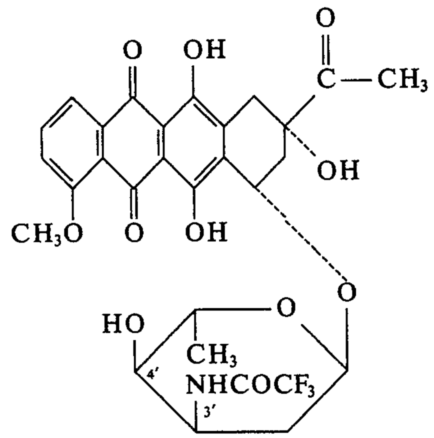

(VIa)

the pure compound (VIa) is separated from this mixture by chromatography, and subsequently the N-trifluoroacetyl group at the sugar moiety is hydrolyzed, thereby obtaining the free glycoside base 3',4'-epi-daunorubicin, which after treatment with methanolic hydrogen halide is isolated as respective hydrohalide (VIII).

3. A process according to claim 1 or 2, characterized in that N-trifluoroacetyl-daunorubicin of the formula

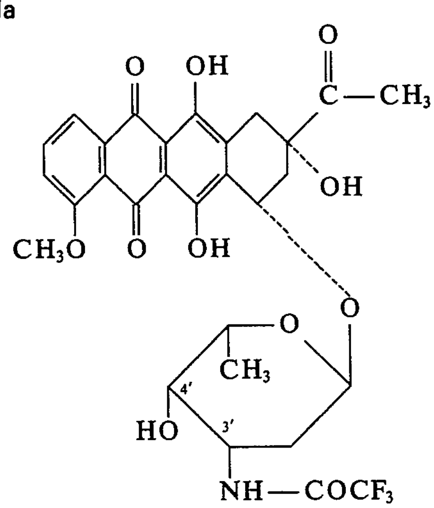

(I)

dissolved in anhydrous methylene chloride, is subjected by treatment with dimethyl sulfoxide, activated with trifluoro-acetic anhydride, to an oxidative reaction of the C-4'-hydroxyl group at a temperature of −65 to −60° C, thereby obtaining after alkalisation of the reaction mixture with a base, such as 1,5-diazo-bicyclo[4,3,o]-non-5-en, the new 4'-keto-N-trifluoroacetyl-daunorubicin of the formula

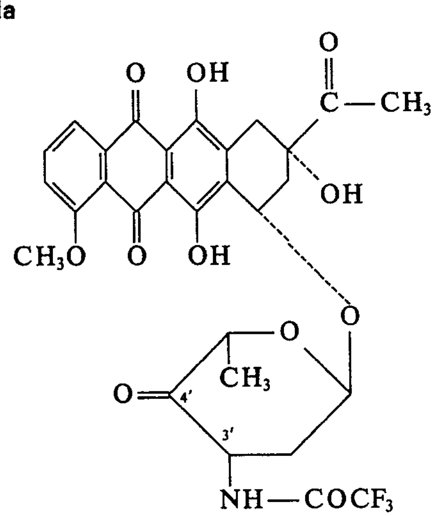

(IIIa)

which, dissolved in methanol, is either subjected to a selective stereo-specific reduction by sodium borohydride at a temperature of −10°C, thereby obtaining 4′-epi-N-trifluoroacetyl-daunorubicin of the formula

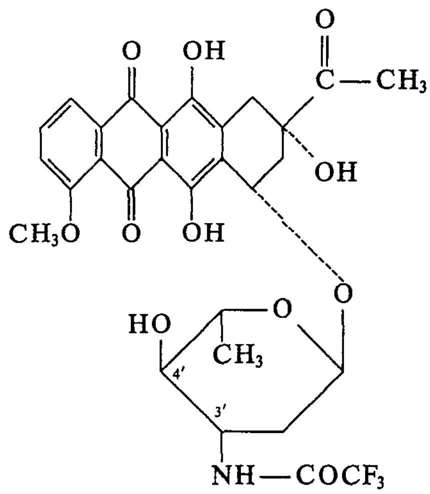

(Va)

from which after mild alkaline hydrolysis of the N-trifluoroacetyl group with 0.1 N aqueous sodium hydroxide the compound 4′-epi-daunorubicin is obtained, and subsequently by treatment with methanolic hydrogen chloride respective hydrochloride (VII) is isolated, or the compound (IIIa), dissolved in chloroform, is filtered through a silica gel column, which is buffered with M/15 phosphate buffer to pH 7, thereby obtaining a 1 : 1 epimeric mixture of the compound (IIIa) and of the new 3′-epi-4′-keto-N-trifluoroacetyl-daunorubicin of the formula

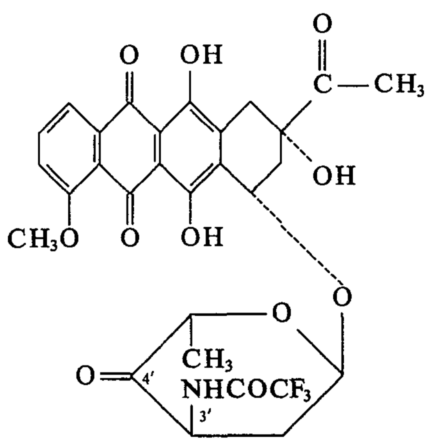

(IVa)

whereupon this epimeric mixture is reduced stereo-specifically with sodium borohydride, thereby obtaining a 1 : 1 mixture of 4′-epi-N-trifluoroacetyl-daunorubicin (Va) and 3′,4′-epi-N-trifluoroacetyl-daunorubicin of the formula

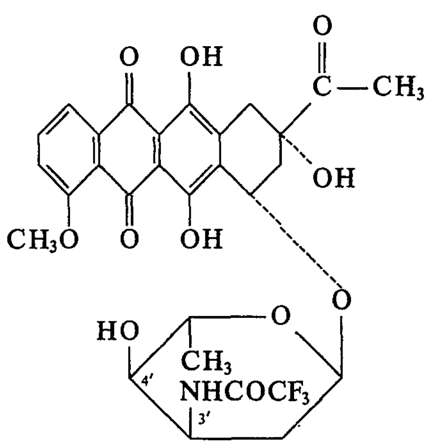

(VIa)

the pure compound (VIa) is separated from this mixture by chromatography on a silical gel column, which is buffered with M/15 phosphate buffer to pH 7, using chloroform/acetone (96 : 4, V/V) as solvent system, and subsequently the N-trifluoroacetyl group at the sugar moiety is hydrolyzed with 0.1 N sodium hydroxide, thereby obtaining the free glycoside base 3',4'-epidaunorubicin, which after treatment with methanolic hydrogen chloride is isolated as respective hydrochloride (VIII).

4. 4'-Eto-N-trifluoroacetyl-daunorubicin.

5. 4'-Keto-N-trichloroethoxycarbonyl-daunorubicin.

6. 3'-Epi-4'-keto-N-trifluoroacetyl-daunorubicin.

7. 3'-Epi-4'-keto-N-trichloroethoxycarbonyl-daunorubicin.

8. The use of the compounds according to one or more of the claims 4 to 7 for the preparation of 4'-epidaunorubicin and 3',4'-diepidaunorubicin.

**Revendications**

1. Procédé pour la préparation de 4'-épidaunorubicine 3',4'-diépidaunorubicine et de leurs halohydrates ayant les formules:

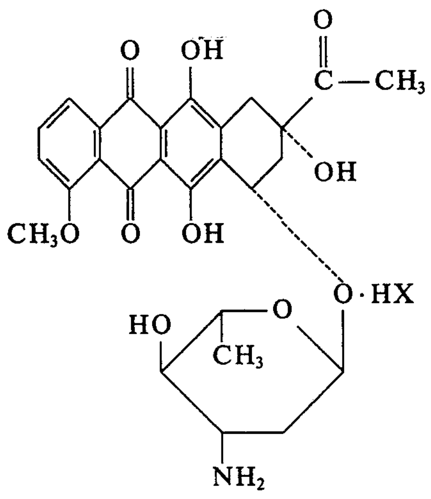

(VII)

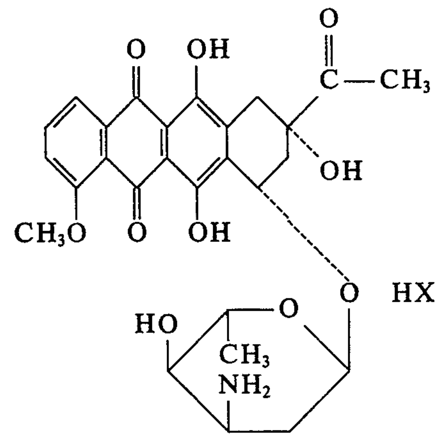

(VIII)

caractérisé par le fait qu'on soumet une daunorubicine à N-protégé ayant la formule:

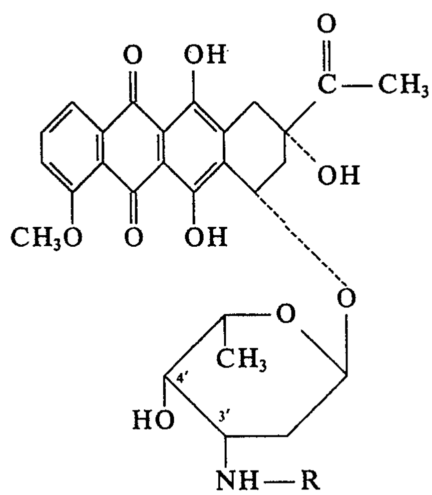

(I)

où R représente un groupe protecteur approprié, dans un solvant anhydre, à une réaction d'oxydation du groupe C-4'-hydroxyle par traitement avec du diméthylsulfoxyde activé par l'anhydride trifluoro-acétique, ce qui fait qu'après avoir rendu basique la solution réactionnelle avec une base, on

obtient une 4'-cétodaunorubicine à N protégé ayant la formule:

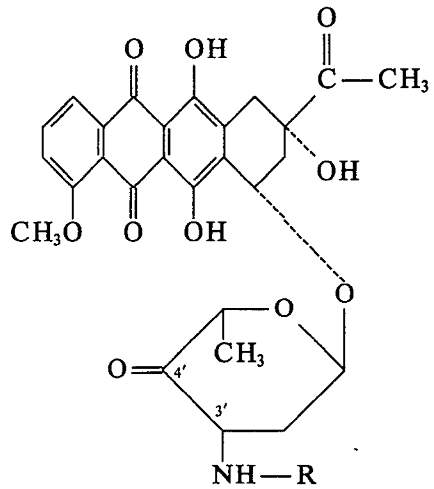

(IIIa)

qu'on fait réagir avec du borohydrure de sodium pour avoir une 4'-épi-daunorubicine à N protégé ayant la formule:

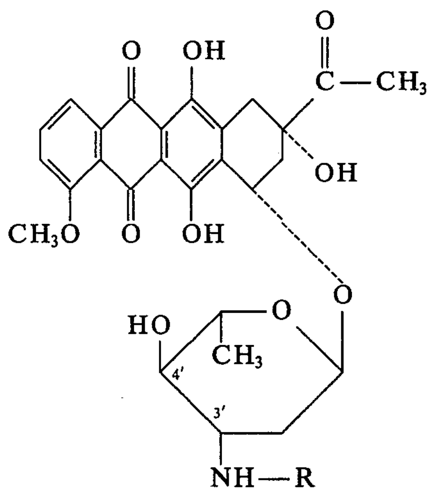

(Va)

à partir de laquelle après hydrolyse alcaline douce du groupe protecteur de N, la 4'-épidaunorubicine est obtenue puis est isolée sous forme de son halohydrate (VII) par traitement avec un hydracide halogéné méthanolique, ou bien le composé (IIIa) dissous dans un hydrocarbure halogéné est filtré à travers une colonne de silicagel tamponné à environ pH 7, ce qui donne un mélange du composé (IIIa) et de 3'-épi-4'-céto-daunorubicine à N protégé ayant la formule:

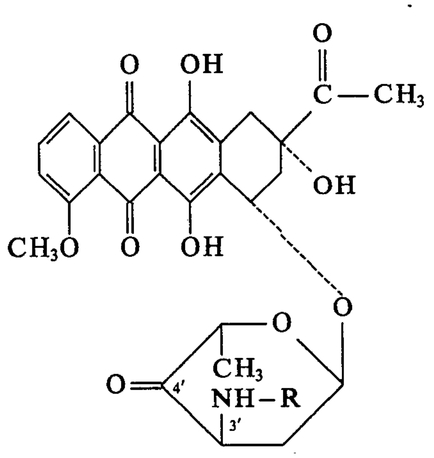

(IVa)

après quoi, ce mélange d'épimères est réduit d'une façon stéréo-spécifique avec du borohydrure de

sodium, ce qui donne un mélange de 4'-épi-daunorubicine à N protégé (Va) et de 3',4'-épi-daunorubicine à N protégé ayant la formule:

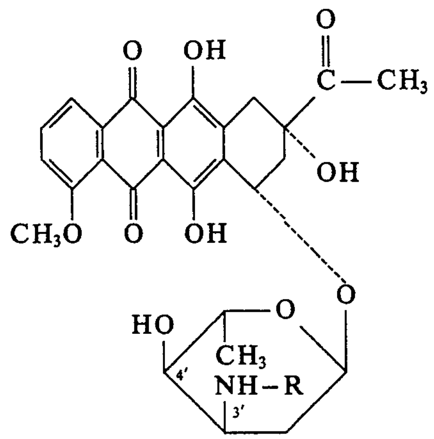

(VIa)

après quoi, le composé pur (VIa) est séparé de ce mélange par chromatographie, puis le groupe protecteur de N sur la partie sucre est hydrolysé, ce qui donne la base glycoside libre 3',4'-épi-daunorubicine qui, par traitement avec un hydracide halogéné méthanolique, est isolée sous forme de l'halohydrate correspondant (VIII).

2. Procédé selon la revendication 1, caractérisé par le fait qu'on soumet la N-trifluoro-acétyldaunorubicine de formule:

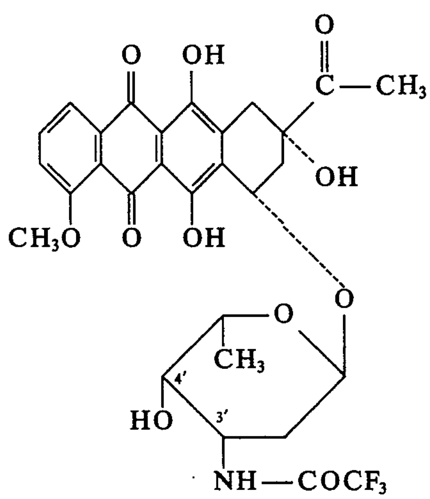

(I)

dans un solvant anhydre, à une réaction d'oxydation du groupe C-4'-hydroxyle par traitement avec du diméthylsulfoxyde activé par l'anhydride trifluoro-acétique, ce qui fait qu'après avoir rendu basique le mélange réactionnel avec une base, on obtient la 4'-céto-N-trifluoro-acétyldaunorubicine ayant la formule:

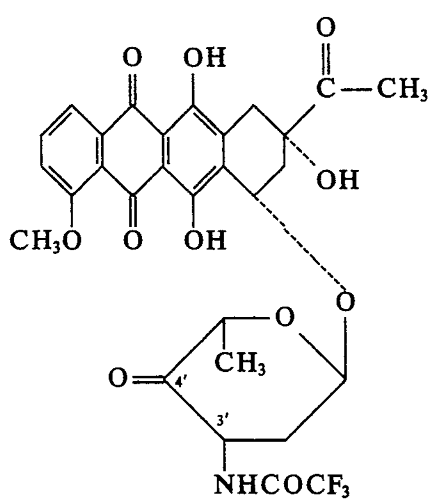

(IIIa)

qu'on fait réagir avec le borohydrure de sodium pour avoir la 4'-épi-N-trifluoro-acétyldaunorubicine ayant la formule:

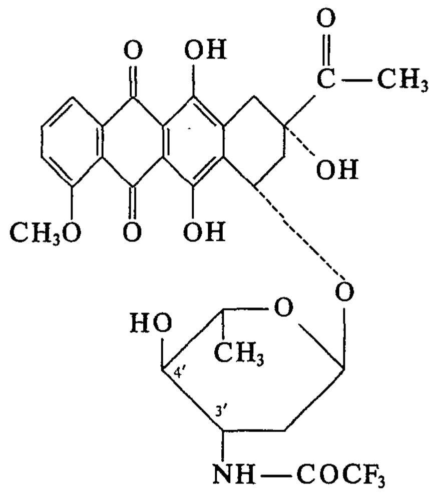

(Va)

à partir de laquelle après hydrolyse alcaline douce du groupe N-trifluoro-acétyle, la 4'-épidaunorubicine est obtenue, puis est isolée sous forme d'halohydrate (VII) par traitement avec un hydracide halogéné méthanolique, ou bien le composé (IIIa) dissous dans un hydrocarbure halogéné est filtré à travers une colonne de silicagel tamponné à environ pH 7, ce qui donne un mélange du composé (IIIa) et de 3'-épi-4'-céto-N-trifluoro-acétyldaunorubicine de formule:

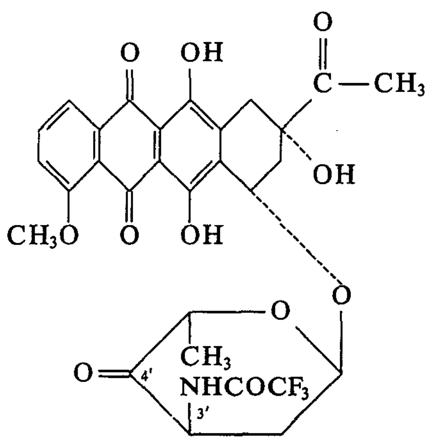

(IVa)

après quoi ce mélange d'épimères est réduit d'une façon stéréo-spécifique avec du borohydrure de sodium, ce qui donne un mélange de 4'-épi-N-trifluoro-acétyldaunorubicine (Va) et de 3',4'-épi-N-trifluoro-acétyldaunorubicine de formule:

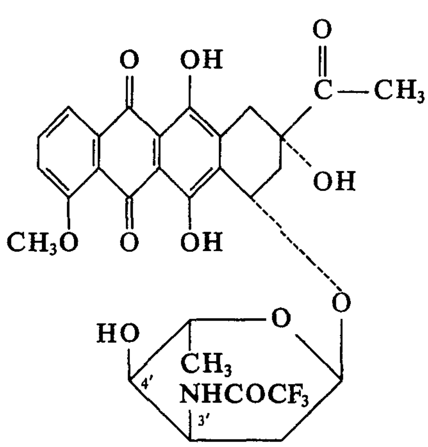

(VIa)

après quoi le composé pur (VIa) est séparé de ce mélange par chromatographie, puis le groupe N-trifluoro-acétyle sur la partie sucre est hydrolysé, ce qui donne la base glycoside libre 3',4'-épidaunorubicine, qui est isolée sous forme de l'halohydrate correspondant (VIII) par traitement avec un hydracide halogéné méthanolique.

3. Procédé selon les revendications 1 ou 2, caractérisé par le fait qu'on soumet la N-trifluoro-acétyldaunorubicine de formule:

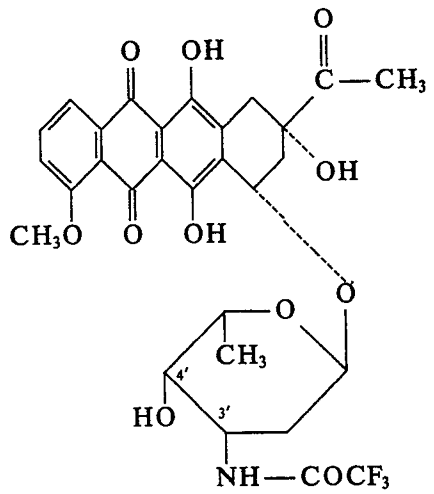

(I)

dissoute dans du chlorure de méthylène anhydre, à une réaction d'oxydation du groupe C-4'-hydroxyle, à une température de −65 à −60°C, par traitement avec du diméthylsulfoxyde activé par l'anhydride trifluoro-acétique, ce qui donne, après avoir rendu basique le mélange réactionnel avec une base, telle que le 1,5-diazobicyclo[4,3,O]-non-5-ène, la nouvelle 4'-céto-N-trifluoro-acétyldaunorubicine de formule:

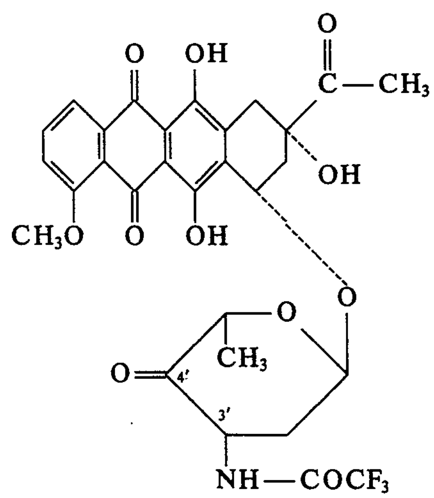

(IIIa)

qui est, soit dissoute dans le méthanol, et soumise à une réduction sélective stéréospécifique, à une température de −10°C, avec du borohydrure de sodium, ce qui donne la 4'-épi-N-trifluoroacétyldaunorubicine de formule:

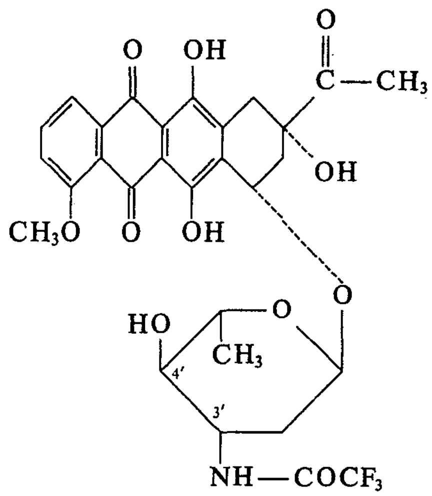

(Va)

à partir de laquelle après hydrolyse alcaline douce du groupe N-trifluoro-acétyle avec de l'hydroxyde de sodium aqueux 0,1 n, la 4'-épidaunorubicine est obtenue, puis est isolée sous forme de chlorhydrate (VII) par traitement avec de l'acide chlorhydrique métanolique, soit que le composé (IIIa) est dissous dans le chloroforme et filtré à travers une colonne de silicagel tamponné à pH 7 avec un tampon phosphate M/15, ce qui donne un mélange d'épimères 1 : 1 du composé (IIIa) et de la nouvelle 3'-épi-4'-céto-N-trifluoro-acétyldaunorubicine de formule:

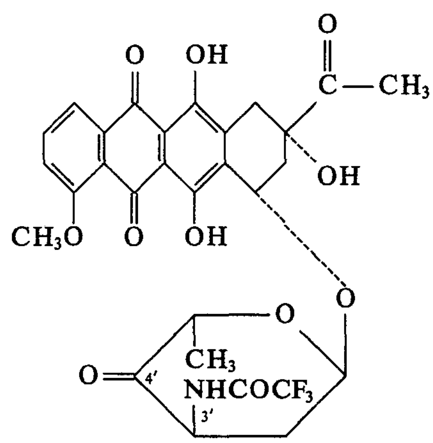

(IVa)

après quoi, ce mélange d'épimères est réduit d'une façon stéréo-spécifique avec du borohydrure de sodium, ce qui donne un mélange 1 : 1 de 4'-épi-N-trifluoro-acétyldaunorubicine (Va) et de 3',4'-épi-N-trifluoro-acétyldaunorubicine de formule:

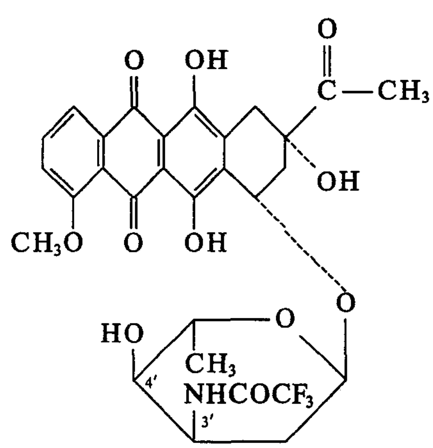

(VIa)

après quoi le composé pur (VIa) est séparé de ce mélange par chromatographie sur une colonne de silicagel tamponné à pH 7 avec un tampon phosphate M/15, en utilisant le système solvant chloroforme-acétone (96 : 4, V/V) puis le groupe N-trifluoro-acétyle de la partie sucre est hydrolysé

par traitement avec de l'hydroxyde de sodium 0,1 n, ce qui donne la base glycoside libre, la 3',4'-épidaunorubicine, qui finalement est isolée sous forme du chlorhydrate correspondant (VIII) par traitement avec de l'acide chlorhydrique méthanolique.

4. 4'-céto-N-trifluoro-acétyldaunorubicine.

5. 4'-céto-N-trichloréthoxycarbonyldaunorubicine.

6. Epi-4'-céto-N-trifluoro-acétyldaunorubicine.

7. 3'-épi-4'-céto-N-trichloréthoxycarbonyldaunorubine.

8. Utilisation des composés selon l'une quelconque des revendications 4 à 7 pour la préparation de 4'-épi-daunorubicine.